# FASCICULE DE BREVET EUROPEEN

(11) **EP 0 333 560 B1**
(45) Date de publication et mention de la délivrance du brevet: **27.04.1994**
(21) Numéro de dépôt: 89400655.0
(22) Date de dépôt: 08.03.1989
(51) Int. Cl.: C12Q 1/02

(54) **Procédé de détermination quantitative de micro-organismes**
Verfahren zur Bestimmung von mikro-organismen
Process for quantitative determination of micro organismes

(30) Priorité: 08.03.1988 FR 8802937
(43) Date de publication de la demande: 20.09.1989
(73) Titulaire: CHEMUNEX, F-94700 Maisons-Alfort (FR)
(72) Inventeur: Van Hoegaerden, Michel, F-77720 Mormant (FR)
(74) Mandataire: Ores, Irène

(56) Documents cités:
- EP-A- 0 122 148
- EP-A- 0 163 206
- EP-A- 0 177 718
- WO-A-86/05206
- JOURNAL OF HISTOCHEMISTRY & CYTOCHEMISTRY, vol. 34, no. 2, February 1986, New York, NY (US); K.D. BAUER et al., pp. 245-250#
- PATENT ABSTRACTS OF JAPAN, vol. 9, no. 145 (C-287)(1868), 20 June1985#
- APPLIED OPTICS, vol. 26, no. 16, 15 August 1987, New York, NY (US); J.W. BACUS et al.; pp. 3280-3293#
- BIOTECHNOLOGY, vol. 3, 1985; pp. 337-356#
- CYTOMETRY, vol. 4; 1983; pp. 222-227#

## Description

La présente invention est relative à un procédé de recherche quantitatif, dans un milieu à l'état liquide, semi-liquide, gélifié ou pâteux, de micro-organismes, même présents en quantité extrêmement faible.

Par quantité extrêmement faible, on entend au sens de la présente invention, un nombre de micro-organismes qui peut être égal à un.

L'analyse qualitative et quantitative rapide de la présence de micro-organismes tels que bactéries, levures, moisissures et parasites dans des produits alimentaires, des produits d'hygiène ou des fluides, ainsi que la surveillance d'un processus de fermentation au cours de sa réalisation, s'avèrent très utiles et importants dans les différentes industries concernées.

On utilise habituellement, comme technique de détection, la mise en culture d'un échantillon du produit à tester. Cette méthode de détection a l'inconvénient d'être longue à mettre en oeuvre, les micro-organismes éventuellement présents se développant dans un délai de 2 à 30 jours. De plus, la numération de micro-organismes par cette méthode n'est absolument pas précise et ne permet qu'une évaluation approximative du nombre de micro-organismes présents au départ ; l'identification des micro-organismes, par cette méthode, reste partielle.

On connaît également une autre technique de détection, par analyse d'images, mais celle-ci, telle que réalisée jusqu'à présent, est coûteuse et difficile à mettre en oeuvre ; en effet, pour arriver à une numération correcte ou une détection sensible de micro-organismes présents dans l'échantillon, il faut analyser tous les champs de l'échantillon, ce qui rend cette recherche très longue.

Il est également connu de marquer des cellules, en particulier des micro-organismes au moyen de colorants chimiques ou biologiques, notamment les colorants des acides nucléiques, qui marquent le plus souvent les cellules mortes et non les cellules vivantes ou les fluorochromes tels que les dérivés de la fluorescéine et de la phycoérythrine, qui sont utilisés depuis de nombreuses années, à travers leur couplage à des anticorps.

L'utilisation d'anticorps polyspécifiques ou monospécifiques rendus fluorescents ont été décrits dans l'Art antérieur, notamment par la Demanderesse dans FR-A-2 598 513 qui vise des anticorps polyspécifiques anti-levures et antimoisissures.

Toutefois, ces anticorps fluorescents rendent fluorescentes toutes les cellules, qu'elles soient vivantes ou mortes, ce qui représente un inconvénient important, soit pour déterminer sélectivement la présence d'un contaminant vivant dans un produit alimentaire ou d'hygiène, soit pour déterminer l'évolution ou la bonne marche d'un processus de fermentation.

D'autres colorants ont également été décrits dans la littérature, en particulier le diacétate de carboxyfluorescéine (CFDA) et le diacétate de fluorescéine (FDA), ce dernier étant utilisé pour la détection des bactéries comme décrit dans un certain nombre d'articles (T.H. CHRZANOWSKI et al., "Applicability of the fluorescein diacetate method of detecting active bacteria in fresh water", paru en 1984 dans Microbial Ecology (10 : 179 - 185), B. Lundgren "Fluorescein diacetate as a stain of metabolically active bacteria in soil", paru dans OIKOS, 1981 (36 : 17 - 22), JARNAGIN et al. "The use of fluorescein diacetate and ethidium bromide as a stain for evaluating viability of mycobacteria" paru dans Stain Technol., 1980, (55, 4, 253 - 258), SCHNURER J. et al. "Fluorescein diactetate hydrolysis as a measure of total microbial activity in soil and litter", paru dans Applied Environm. Microbiol., 1982, (43, 6, 1256 - 1261), SONTAG qui décrivent ces colorants et leur mécanisme d'action. Toutefois, il s'agit en général de méthodes d'estimation, ou bien, lorsque la méthode est quantitative, elle utilise en association un colorant chimique des acides nucléiques ; ce sont des méthodes peu appropriées à l'analyse en série.

Les techniques de détection selon l'Art antérieur ne permettent pas la détection sélective de micro-organismes vivants, surtout présents en très petite quantité.

WO-A-8 605 206 décrit par exemple une méthode d'évaluation de la concentration de microorganismes présents dans un échantillon par rapport à une population standard : impliquant l'induction d'une fluorescence par clivage, à l'aide d'une enzyme propre aux microorganismes, d'un colorant non fluorescent ajouté préalablement et la mesure de la fluorescence totale émise par l'emsemble des microorganismes, cette méthode ne permet pas de détecter des concentrations inférieures à 10² cellules/ml.

En conséquence, l'invention s'est donnée pour but de pourvoir à un procédé de recherche, de détection et de numération de micro-organismes, très rapide et facile à mettre en oeuvre, sensible, spécifique et peu coûteux, permettant le comptage à l'unité près desdits micro-organismes.

La présente invention a pour objet un procédé de détermination quantitative de microorganismes, présents normalement, ou éventuellement contenus en tant que contaminants dans un milieu complexe à l'état liquide, semi-liquide, gélifié ou pâteux, notamment des produits alimentaires, des produits d'hygiène ou des fluides biologiques, lequel procédé comprend des étapes d'obtention d'échantillon et d'addition d'un marqueur de viabilité positive apte à marquer des microorganismes vivants, choisi parmi les substances non fluorescentes ou peu fluorescentes, qui pénètrent dans le microorganisme, lesdites substances étant alors clivées ou modifiées de manière à fournir un produit fluorescent, ledit produit étant piégé dans sa majeure partie ou totalement à l'intérieur dudit micro-organisme, lequel procédé est caractérisé en ce qu'il comprend :
A. pour le marquage spécifique et sélectif des microorganismes:
   a) la séparation desdits microorganismes par filtration et/ou centrifugation d'une suspension d'échantillon, obtenue directement lorsque l'échantillon de départ est liquide, ou obtenue par la mise en contact de l'échantillon avec un tampon d'ajustement du pH du milieu à un pH alcalin, dans le cas où l'échantillon de départ est semi-liquide ou pâteux ;
   b) le marquage intracellulaire desdits microorganismes par incubation avec au moins un marqueur de viabilité positive pendant 5 à 10 min à 37°C,
   c) le traitement de l'échantillon obtenu en b) aux ultrasons, pendant 10 s, pour individualiser les microorganismes, et
B. pour le dénombrement desdits microorganismes rendus fluorescents :
   d) le passage de l'échantillon obtenu en c) dans un cytomètre en flux, et
   e) le comptage à l'aide dudit cytomètre en flux de chaque microorganisme marqué par la mesure de la/les fluorescences intracellulaires émises par chacun desdits microorganismes.

On entend par micro-organismes, au sens de la présente invention, notamment des bactéries, des levures, des moisissures et des parasites.

On entend par marqueur de viabilité positive, une substance qui ne colore que les cellules vivantes et non les cellules mortes et dont l'intensité de coloration des cellules est proportionnelle à leur activité métabolique.

Les colorants vitaux, au sens de la présente invention, sont des colorants des acides nucléiques et se combinent avec le matériel génétique des cellules aussi bien vivantes que mortes.

On peut citer, à titre d'exemple non limitatif de colorants vitaux, le bromure d'éthydium, l'iodure du propydium, la mythramycine A, le DAPI, le colorant commercialisé sous le nom Hoechst 33258, le colorant commercialisé sous le nom Hoechst 33342.

La cytomètrie en flux, en soi connue est décrite dans de nombreux documents en particulier dans EP-A-177 718.

La combinaison de moyens selon l'invention, a l'avantage de permettre la réalisation de dosages en série, rapides à mettre en oeuvre, fiables ainsi que des comptages à l'unité près. Il ne s'agit pas d'une méthode d'évaluation de concentration par rapport à une population standard, il n'est donc pas nécessaire de connaître l'espèce ou le genre du microorganisme avant l'analyse.

Selon un mode de mise en oeuvre avantageux dudit procédé, le tampon d'ajustement du pH est utilisé en tant que milieu de solubilisation.

Selon encore un autre mode de mise en oeuvre avantageux dudit procédé, le traitement préalable comprend en outre l'action d'enzymes appropriées.

Le traitement (avant et après le marquage) permet l'individualisation des cellules, formant à l'origine des colonies, et augmente ainsi la sensibilité de la détection.

Le marqueur de viabilité positive est choisi parmi les substances non fluorescentes ou peu fluorescentes, qui pénètrent dans le micro-organisme, lesdites substances étant alors clivées ou modifiées de manière à fournir un produit fluorescent, ledit produit étant piégé dans sa majeure partie ou totalement à l'intérieur dudit micro-organisme.

Les marqueurs de viabilité, conformes à l'invention, sont choisis dans le groupe qui comprend le FDA, le 6-CFDA, le 5-CFDA, le dilaurate de fluorescéine, l'ester de 5,6-CFDA N-hydroxy succinimide, le dipropionate de fluorescéine, la di-bêta-D-galactoside fluorescéine, le phosphate de 3-O-méthylfluorescéine, le pentaacétoxy ester de 2',7'-bis(carboxyéthyl)-5(6)-carboxyfluorescéine (BCECF/AM), le diacétate d'azidofluorescéine, l'acétate de 4-méthylumbelliferyl, le 4-méthylumbelliferyl bêta-D-galactoside, le 4-méthylumbelliferyl alpha-D-mannopyranoside, le nonanoate de 4-méthyllumbelliferyl, le phosphate de 4-méthylumbelliferyl, l'alanine-7-amino-4-méthylcoumarine, la glycine-7-amino-4-méthylcoumarine, la proline-7-amino-4-méthylcoumarine, la valine-7-amino-4-méthylcoumarine, la glycyl-L-proline-7-amino-4-méthylcoumarine, le 1,4-diacétoxy-2,3-dicyanobenzène (ABD), l'hydroéthidine, l'acétate de résorufine.

Selon un autre mode de mise en oeuvre avantageux dudit procédé, lesdits microorganismes sont en outre soumis à un marquage additionnel avec au moins une substance choisie dans le groupe constitué par d'autres marqueurs de viabilité positive, des colorants vitaux, des anticorps rendus fluorescents et des sondes nucléiques rendues fluorescentes.

Conformément à ce dernier mode de mise en oeuvre avantageux dudit procédé, les anticorps sont choisis parmi les anticorps polyclonaux et/ou monoclonaux, mono ou polyspécifiques, anti-levures et/ou anti-bactéries et/ou anti-moisissures et/ou anti-parasites et les anticorps polyclonaux et/ou monoclonaux mono ou polygénériques anti-levures et/ou anti-bactéries et/ou anti-moisissures et/ou anti-parasites.

Conformément à l'invention, les anticorps mis en oeuvre sont rendus fluorescents par un agent fluorochrome approprié.

Les marqueurs de viabilité ont pour rôle de marquer sélectivement les micro-organismes vivants.

Les anticorps ont pour rôle de marquer spécifiquement une espèce ou un genre de micro-organisme, que celui-ci soit vivant ou mort.

Le procédé de détermination conforme à l'invention peut avantageusement être mis en oeuvre dans une installation comprenant un cytomètre en flux à cellule de mesure comprenant une chambre de mesure en flux que traverse l'échantillon de produit ou de fluide à analyser et un dispositif d'enregistrement des signaux de fluorescence émis par lesdits micro-organismes, la cellule de mesure étant pourvue d'un dispositif de rinçage de la chambre de mesure, lequel entre en action sous l'effet du retrait de l'échantillon du porte-échantillon ou sous l'effet d'une commande électronique externe appropriée.

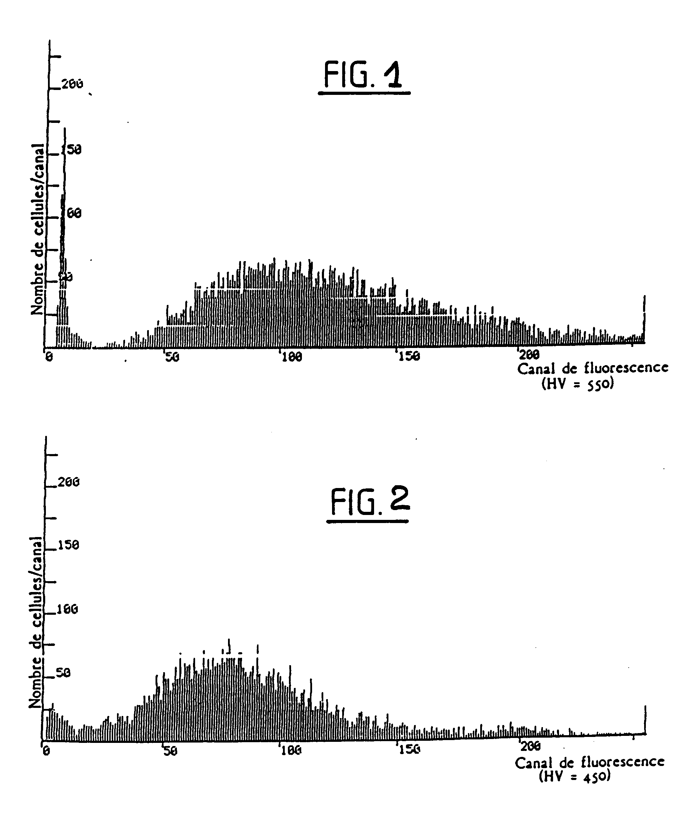

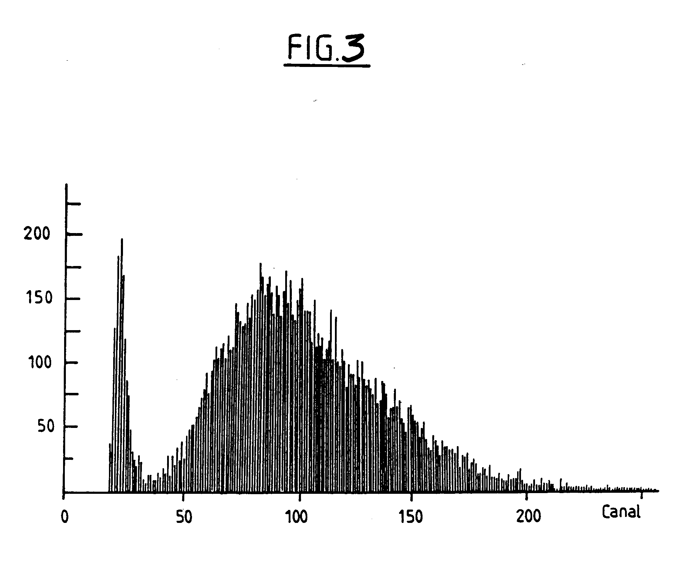

Outre les dispositions qui précèdent, l'invention comprend encore d'autres dispositions, qui ressortiront de la description qui va suivre, qui se réfère à des exemples de mise en oeuvre du procédé conforme à l'invention, avec référence aux figures 1 à 12 sont des diagrammes.

Il doit être bien entendu, toutefois, que les exemples, les dessins et les parties descriptives correspondantes sont donnés uniquement à titre d'illustration de l'objet de l'invention, dont ils ne constituent en aucune manière une limitation.

**Exemple 1** : Recherche de levures contaminant le yaourt.

Il est possible, selon la/les espèce(s) de levure à rechercher, de choisir le marqueur de viabilité et/ou les anticorps anti-levures les plus appropriés à ladite levure.

Le yaourt est solubilisé au moyen de réactifs appropriés, tel que le tampon carbonate EDTA pH 9,0. Le produit est centrifugé 5 minutes à 1 500 g. Le culot est repris dans du chlorure de sodium à 9 g/l. Le diacétate de fluorescéine, éventuellement associé aux anticorps fluorescents anti-levures appropriés sont rajoutés. Une incubation de 5 à 10 minutes à 37°C permet aux réactions chimiques de se produire ; la suspension est soumise aux ultra-sons pendant 10 secondes. La suspension résultante est ensuite passée directement dans le compteur de particules pour une analyse continue en flux, sous un volume variant de 0,2 à 2 ml. Il est possible de compter ou dénombrer significativement à l'unité près, les micro-organismes rendus fluorescents (figures 1, 2 et 3).

Les figures 1, 2 et 3 représentent des histogrammes de population de levures cultivées dans le yaourt.

On a en abscisse le canal de fluorescence représentant l'intensité de lumière et en ordonnée le nombre de cellules par canal.

Dans le cas de la figure 1, il s'agit de levures du genre *Saccharomyces* ; dans le cas de la figure 2, il s'agit de levures du genre *Rhodotorula* et dans le cas de la figure 3, il s'agit de *Debaryomyces hansenii*.

**Exemple 2** : Recherche de levures contaminant le yaourt à pulpe de fruits :
Dans le cas où l'échantillon est consitué de yaourts à pulpe de fruits, des enzymes sont ajoutées pour favoriser la solubilisation dudit échantillon.

L'échantillon est traité comme dans l'exemple 1, sauf que préalablement à l'addition du colorant et/ou de l'anticorps, de la pectinase est rajoutée pour favoriser la solubilisation ; la réaction enzymatique s'effectue en incubant à 37°C et pendant 5 minutes l'échantillon de yaourt en présence de l'enzyme.

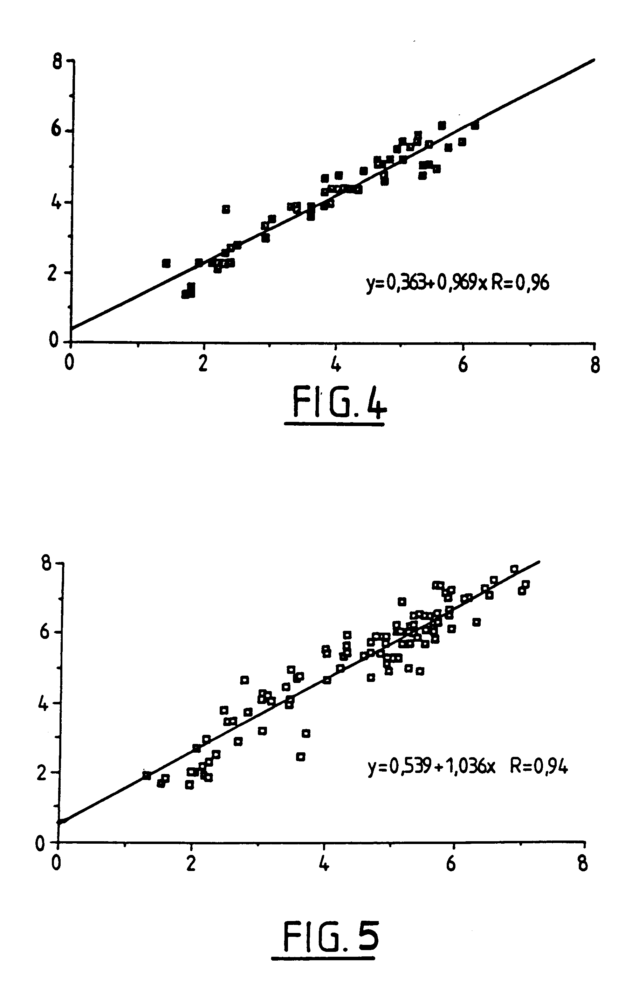

Les figures 4 (yaourt nature) et 5 (yaourt aux fruits) montrent des résultats comparatifs entre le procédé conforme à l'invention et la méthode classique de culture en boîtes de Pétri.

Ces figures comportent en abscisses les résultats obtenus par le procédé conforme à l'invention (log (coups/ml)) et en ordonnées les résultats obtenus par la méthode classique (log (levures/ml)). On observe une très bonne corrélation entre les deux méthodes.

**Exemple 3** : Recherche de bactéries dans la bière ou dans des milieux de culture servant à la fermentation d'organismes recombinés (production de médicaments par exemple).

Typiquement, 50 ml de suspension cellulaire sont centrifugés. Le culot est remis en suspension tel qu'indiqué dans l'exemple 1. La suite du procédé est similaire, les marqueurs de viabilité ou les anticorps étant toutefois adaptés au problème spécifique. Cependant, le marqueur de viabilité ayant une fluorescence dans le vert, les anticorps sont marqués au moyen d'un fluorochrome à émission dans le rouge, la phycoérythrine par exemple ; il est alors possible, dans ce cas, d'effectuer un comptage des micro-organismes doublement marqués, ce qui permet d'identifier le micro-organisme et de déterminer simultanément sa viabilité et sa vitalité.

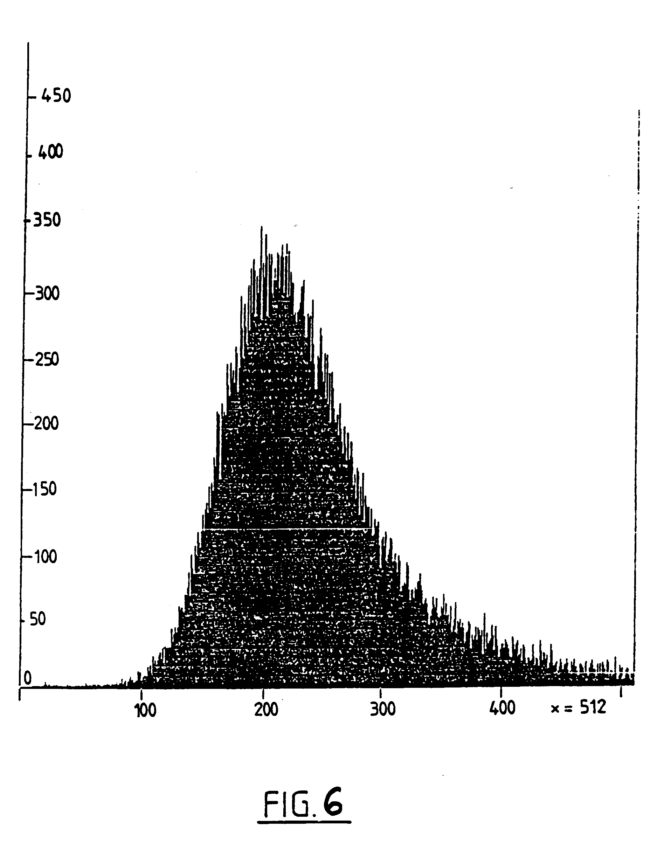

La figure 6 représente un histogramme de population de bactéries du genre *Lactobacillus* détecté dans un échantillon de bière naturellement contaminé. En abscisse on a le canal de fluorescence, et en ordonnée le nombre de particules par canal. Les particules les plus fluorescentes sont celles qui présentent un métabolisme très actif.

**Exemple 4** : Recherche de bactéries et/ou de levures dans des produits préparés (exemple pâte de tomates, mayonnaise, sauce béarnaise, etc...)
Après une extraction aqueuse de ces produits, la suspension résultante est filtrée grossièrement à travers un filtre de 15 µm par exemple. Le produit filtré est centrifugé ainsi qu'indiqué dans l'exemple 1 ci-dessus. Le reste du processus est similaire, ayant toutefois la possibilité d'utiliser en mélange deux marqueurs de viabilité ayant une fluorescence dans le vert et deux anticorps monoclonaux ayant une fluorescence dans le rouge à des longueurs d'onde différentes, par exemple. L'appareil comprendra alors une mesure des 3 paramètres, permettant de distinguer et de compter ou dénombrer de façon précise à l'unité prés, simultanément les levures vivantes d'une espèce donnée, les levures mortes de même espèce, les bactéries vivantes d'une espèce donnée, les bactéries mortes de même espèce, le total des levures vivantes d'autres espèces et le total des bactéries vivantes d'autres espèces.

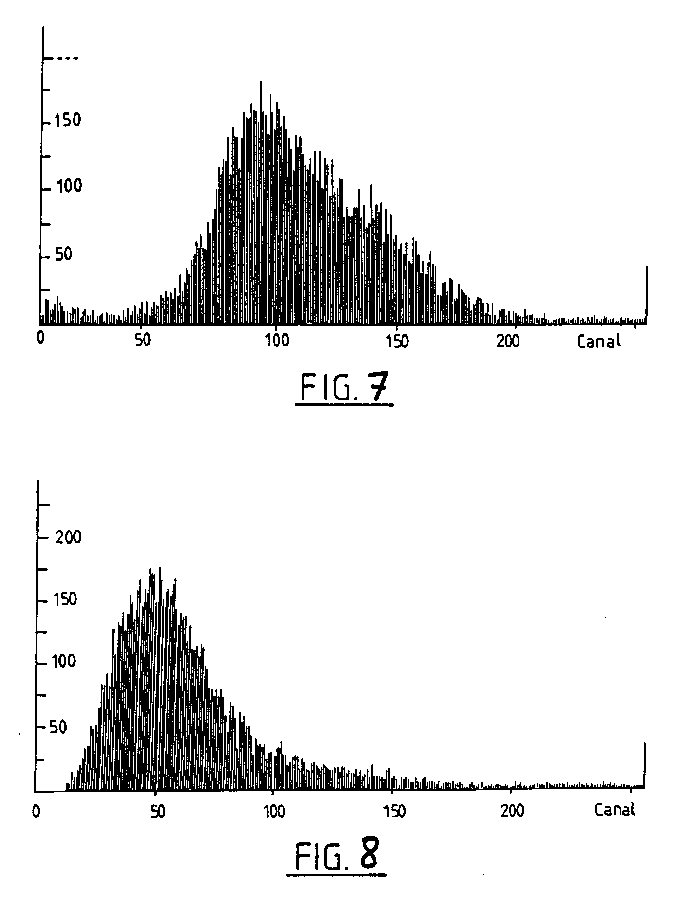

La figure 7 représente un histogramme d'une population de *Candida parapsilosis* contaminant une sauce béarnaise. En abscisse, on a le canal de fluorescence, et en ordonnée le nombre de micro-organismes par canal.

**Exemple 5** : Recherche de bactéries totales dans le lait cru.

Le lait cru est traité pour détruire les micelles de caséine et est ensuite centrifugé ; le culot est repris dans un tampon tel que décrit dans l'exemple 1, en ajoutant en plus quelques gouttes d'un détergent approprié tel que Tween® 20. La suite du processus est similaire. Toutefois, les cellules somatiques du lait peuvent se colorer. On procède alors à une mesure et un comptage simultanés de la fluorescence et de la taille des particules par impédancemétrie, ce qui permet de différencier les bactéries (moins de 1 mm en taille) des cellules somatiques (environ 10 µm ou plus en taille).

**Exemple 6** : Recherche de bactéries pathogènes dans les liquides biologiques humains, tel le liquide céphalo-rachidien ou l'urine.

La détection précoce d'une méningite à méningocoques peut être réalisée directement à partir du fluide cérébro-spinal par centrifugation, et coloration du culot par anticorps monoclonaux spécifiques disponibles commercialement. La culture du micro-organisme spécifique n'est plus nécessaire, la sensibilité de l'appareil étant suffisante. Le marqueur de viabilité positive est utilisé simultanément pour s'assurer de l'état métabolique des cellules. Similairement, les bactéries (bactériémie) ou les parasites peuvent être recherchés dans les urines, par exemple.

La figure 8 illustre la détection et la numération d'entérocoques dans un échantillon d'urine.

**Exemple 7** : Numération rapide de levures dans un brassin :
La suspension de levures constituant le brassin est diluée dans du NaCl à 9 g/l de manière à obtenir une densité de cellules comprise entre 5 000 et 50 000/ml. Une solution du marqueur de viabilité adéquat est ajoutée à une concentration de 1 %. Une incubation de 5 mn à 45°C colore les levures, que l'on peut ensuite compter ou dénombrer de façon précise à l'unité près, au moyen de l'installation conforme à l'invention, en moins d'une minute.

**Exemple 8** : Numération rapide de levures dans un levain :
On procède comme dans l'exemple 7.

**Exemple 9** : Test de germination de spores de moisissures et numération rapide des spores ayant germé :
Les moisissures utiles sont généralement fournies lyophilisées aux industries fromagères. Leur germination et le délai d'obtention de celle-ci peuvent être déterminés en les plaçant dans un tampon de germination favorable au gonflement de leur paroi, notamment solution saline tamponnée au phosphate et additionnée d'une substance énergétique telle que glucose, saccharose, fructose, etc... ou, éventuellement, milieu alimentaire.

Après 4 à 6 heures d'incubation, le gonflement de la paroi des spores rend celles-ci perméables aux marqueurs de viabilité, utilisé de la même manière que dans l'exemple 7.

L'analyse de l'échantillon dans l'installation conforme à l'invention, permet, après dilution adéquate, dans un délai de l'ordre d'une minute, de déterminer le pourcentage de spores germées et leur nombre total.

**Exemple 10** : Suivi d'une mise en culture d'une population de levures, et de la fermentation qui en résulte.

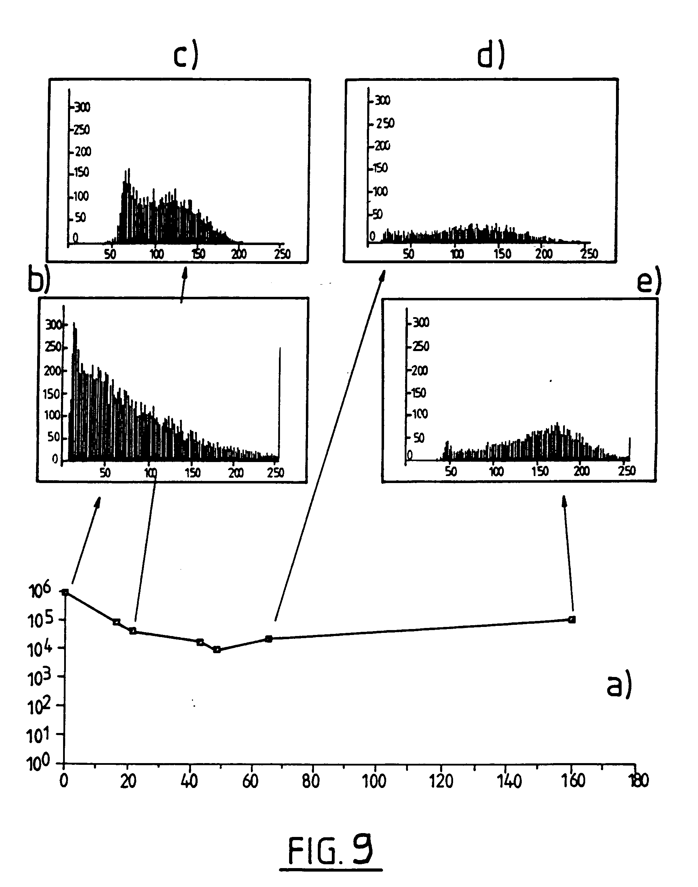

Une population de levures est inoculée dans un moût de bière à une concentration de 10⁶ cellules/ml. Une solution de marqueur de viabilité, marquant spécifiquement les levures est ajoutée à une concentration de 1 %. Une incubation de 5 min à 45°C colore les levures que l'on peut ensuite compter, à l'aide d'un cytomètre en flux approprié. Dans ce cas, on peut déterminer d'une part, que le nombre de cellules vivantes diminue, et que d'autre part l'activité métabolique moyenne de la population augmente (intensité moyenne de fluorescence sur les histogrammes) : l'activité globale de la population augmente (fermentation réussie) même si le nombre de cellules n'augmente pas (chiffre donné par les méthodes classiques) (figure 9).

La figure 9 montre l'évolution de la viabilité et de la vitalité d'une population de levures (*Sacchoromyces cerevisiae*) dans un moût de bière à 10°-12°C au cours du temps.

La figure 9a est une courbe d'évolution du nombre de cellules au cours du temps. Un prélèvement et un comptage sont réalisé au temps 0 (figure 9b), au bout de 20 h (figure 9c), au bout de 60 h (figure 9d) et au bout de 160 h (figure 9e). On voit que le nombre de cellules diminue au cours du temps, alors que les histogrammes sont déplacés vers la droite, ce qui montre un accroissement de la vitalité des cellules présentes.

**Exemple 11** : Suivi de l'effet d'un traitement thermique sur une population de levures en croissance dans un moût de bière.

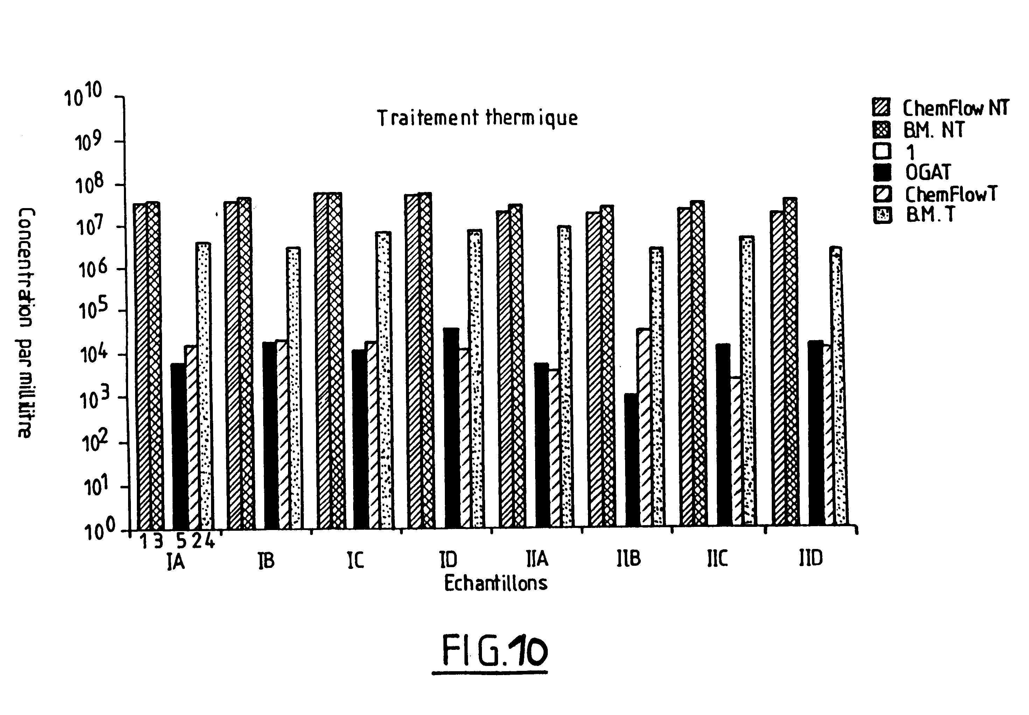

Une population de levures est soumise à un choc thermique à 60°C pendant 5 minutes (figure 10). Cette figure 10 comporte, en abscisse différentes souches de *Saccharomyces cerevisiae* (A, B, C, D), I et II représentant deux moûts de bière différents, et en ordonnée, la concentration cellulaire/ml.

Les populations sont analysées avant et après le traitement : procédé conforme à l'invention : avant traitement : colonne 1 ; après traitement : colonne 2 (réponse en 15 minutes) ; la méthode au bleu de méthylène : avant traitement : colonne 3 ; après traitement : colonne 4 (réponse en environ 30 minutes) et méthode de culture sur boîtes de Pétri : colonne 5 (réponse en 3 jours). On constate que la correspondance procédé selon l'invention-bleu de méthylène est satisfaisante avant traitement thermique mais pas après ce traitement, la référence utilisée étant la méthode de culture sur boîtes. Le résultat est obtenu dans un délai considérablement réduit.

**Exemple 12** : Recherches de levures et de bactéries dans le jus d'orange.

Les marqueurs de viabilité spécifiques respectivement des bactéries et des levures sont ajoutés simultanément au culot de centrifugation de 10 ml de jus d'orange pulpeux.

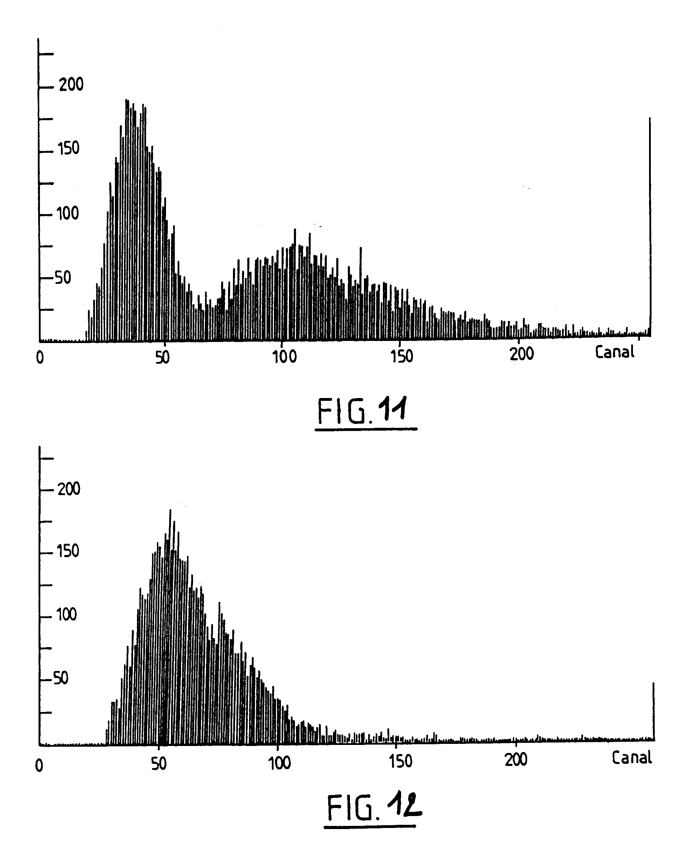

Après une incubation de 10 minutes à 40°C, l'échantillon est passé dans le cytomètre conforme à l'invention. La figure 11 illustre le résultat d'une analyse dans laquelle le pic de gauche représente la population de bactéries et le pic de droite la population de levures. L'appareil permet de déterminer les nombres totaux de cellules dans chaque pic (figure 11).

**Exemple 13** : Recherche d'une contamination bactérienne dans un produit cosmétique.

L'on procède à un traitement et une coloration similaires à ce qui est décrit précédemment. La figure 12 illustre le résultat obtenu avec un produit contaminé, alors que lorsque le produit n'est pas contaminé, on obtient aucun signal de fluorescence, donc un résultat négatif.

## Revendications

1. Procédé de détermination quantitative de microorganismes, présents normalement, ou éventuellement contenus en tant que contaminants dans un milieu complexe à l'état liquide, semi-liquide, gélifié ou pâteux, notamment des produits alimentaires, des produits d'hygiène ou des fluides biologiques, lequel procédé comprend des étapes d'obtention d'échantillon et d'addition d'un marqueur de viabilité positive apte à marquer des microorganismes vivants, choisi parmi les substances non fluorescentes ou peu fluorescentes, qui pénètrent dans le microorganisme, lesdites substances étant alors clivées ou modifiées de manière à fournir un produit fluorescent, ledit produit étant piégé dans sa majeure partie ou totalement à l'intérieur dudit micro-organisme, lequel procédé est caractérisé en ce qu'il comprend :
A. pour le marquage spécifique et sélectif des microorganismes:
a) la séparation desdits microorganismes par filtration et/ou centrifugation d'une suspension d'échantillon, obtenue directement, lorsque l'échantillon de départ est liquide, ou obtenue par la mise en contact de l'échantillon avec un tampon d'ajustement du pH du milieu à un pH alcalin, dans le cas où l'échantillon de départ est semi-liquide ou pâteux ;
b) le marquage intracellulaire desdits microorganismes par incubation avec au moins un marqueur de viabilité positive pendant 5 à 10 min à 37°C,
c) le traitement de l'échantillon obtenu en b) aux ultrasons, pendant 10 s, pour individualiser les microorganismes, et
B. pour le dénombrement desdits microorganismes rendus fluorescents :
d) le passage de l'échantillon obtenu en c) dans un cytomètre en flux, et
e) le comptage à l'aide dudit cytomètre en flux de chaque microorganisme marqué par la mesure de la/les fluorescences intracellulaires émises par chacun desdits microorganismes.

2. Procédé selon la revendication 1, caractérisé en ce que le tampon d'ajustement du pH est utilisé en tant que milieu de solubilisation.

3. Procédé selon la revendication 1, caractérisé en ce que le traitement préalable comprend en outre l'action d'enzymes appropriées.

4. Procédé selon la revendication 1, caractérisé en ce que lesdits microorganismes sont en outre soumis à un marquage additionnel avec au moins une substance choisie dans le groupe constitué par d'autres marqueurs de viabilité positive, des colorants vitaux, des anticorps rendus fluorescents et des sondes nucléiques rendues fluorescentes.

5. Procédé selon la revendication 4, caractérisé en ce que les anticorps sont choisis parmi les anticorps polyclonaux et/ou monoclonaux, mono ou polyspécifiques, anti-levures et/ou anti-bactéries et/ou anti-moisissures et/ou anti-parasites et les anticorps polyclonaux et/ou monoclonaux mono ou polygénériques anti-levures et/ou anti-bactéries et/ou anti-moisissures et/ou anti-parasites.

6. Procédé selon la revendication 5, caractérisé en ce que les anticorps mis en oeuvre sont rendus fluorescents par un agent fluorochrome approprié.

## Claims

1. Process for quantifying microorganisms which are normally present or may be contained as contaminants in a complex medium in the liquid, semi-liquid, gelled or pasty state, in particular food products, hygiene products or biological fluids, this process comprising steps of obtaining a sample and of adding a positive viability marker capable of marking living microorganisms and chosen from the non-fluorescent or low-fluorescence substances, which penetrate into the microorganism, the said substances then being cleaved or modified so as to provide a fluorescent product, the said product being trapped for the most part or in total within the said microorganism, the said process being characterized in that it comprises:
A. for the specific and selective marking of the microorganisms:
a) separating the said microorganisms by filtration and/or centrifuging of a sample suspension, obtained directly when the starting sample is liquid, or obtained by bringing the sample into contact with a buffer for adjusting the pH of the medium to an alkaline pH, where the starting sample is semi-liquid or pasty;
b) intracellular marking of the said microorganisms by incubating with at least one positive viability marker for 5 to 10 minutes at 37°C,
c) treating the sample obtained in b) with ultrasound for 10 s to distinguish the microorganisms, and
B. for counting the said microorganisms which have been made fluorescent:
d) passing the sample obtained in c) through a flow cytometer, and
e) counting, with the aid of the said flow cytometer, each marked microorganism by measuring the intracellular fluorescence(s) emitted by each of the said microorganisms.

2. Process according to Claim 1, characterized in that the pH-adjusting buffer is used as a solubilizing medium.

3. Process according to Claim 1, characterized in that the previous treatment moreover comprises the action of appropriate enzymes.

4. Process according to Claim 1, characterized in that the said microorganisms moreover undergo additional marking with at least one substance selected from the group comprising other positive viability markers, vital stains, antibodies which have been made fluorescent and nucleus probes which have been made fluorescent.

5. Process according to Claim 4, characterized in that the antibodies are selected from polyclonal and/or monoclonal, mono- or polyspecific, anti-yeast and/or anti-bacterial and/or anti-fungal and/or anti-parasitic antibodies, and polyclonal and/or monoclonal mono- or polygeneric anti-yeast and/or anti-bacterial and/or anti-fungal and/or anti-parasitic antibodies.

6. Process according to Claim 5, characterized in that the antibodies used are made fluorescent by an appropriate fluorochrome.

## Patentansprüche

1. Verfahren zur quantitativen Bestimmung von Mikroorganismen, die normalerweise vorhanden sind oder gegebenenfalls als Verunreinigungen in einem komplexen Medium in flüssigem, halbflüssigem, geliertem oder pastösen Zustand, insbesondere in Lebensmittelprodukten, Hygieneprodukten oder biologischen Flüssigkeiten, vorhanden sind, wobei das Verfahren die Stufen Gewinnung der Probe und Zugabe eines positiven Lebensfähigkeitsmarkers mit der Eignung, lebende Mikroorganismen zu markieren, ausgewählt aus nicht-fluoreszierenden oder wenig fluoreszierenden Substanzen, die in den Mikroorganismus eindringen, wobei die Substanzen dann so gespalten oder modifiziert werden, daß sie ein fluoreszierendes Produkt ergeben, welches dann in seinem Hauptteil oder vollständig im Inneren des Mikroorganismus eingeschlossen wird, unfaßt, dadurch **gekennzeichnet**, daß man
A. zur spezifischen und selektiven Markierung der Mikroorganismen
a) die Mikroorganismen durch Filtration und/oder Zentrifugation einer im Falle einer flüssigen Ausgangsprobe direkt erhaltenen oder im Falle einer halbflüssigen oder pastösen Ausgangsprobe durch Inkontaktbringen der Probe mit einem Puffer zur Einstellung des pH-Werts des Mediums auf einen alkalischen pH-Wert erhaltenen Probensuspension abtrennt;
b) intrazellulär die Mikroorganismen durch fünf- bis zehnminütige Inkubation mit mindestens einem positiven Lebensfähigkeitsmarker bei 37°C markiert;
c) die in b) erhaltene Probe mit Ultraschall zehn Sekunden lang behandelt, um die Mikroorganismen zu vereinzeln; und
B. zur Zählung der fluoreszierend gemachten Mikroorganismen
d) die in c) erhaltene Probe durch ein Durchflußzytometer leitet; und
e) mit Hilfe des Durchflußzytometers jeden markierten Mikroorganismus zählt, indem man die intrazelluläre(n) Fluoreszenz(en), die von jedem der Mikroorganismen emittiert wird bzw. werden, mißt.

2. Verfahren nach Anspruch 1, dadurch **gekennzeichnet**, daß der Puffer zur Einstellung des pH-Werts als Medium zur Solubilisation verwendet wird.

3. Verfahren nach Anspruch 1, dadurch **gekennzeichnet**, daß die Vorbehandlung weiterhin die Wirkung geeigneter Enzyme umfaßt.

4. Verfahren nach Anspruch 1, dadurch **gekennzeichnet**, daß die Mikroorganismen weiterhin zusätzlich mit mindestens einer Substanz ausgewählt aus der Gruppe bestehend aus anderen positiven Lebensfähigkeitsmarkern, Vitalfarbstoffen, fluoreszierend gemachten Antikörpern und fluoreszierend gemachten Nucleinsäuresonden markiert werden.

5. Verfahren nach Anspruch 4, dadurch **gekennzeichnet**, daß die Antikörper aus polyclonalen und/oder monoclonalen, mono- oder polyspezitischen, Anti-Hefen- und/oder Anti-Bakterien- und/oder Anti-Schimmelpilze- und/oder Anti-Parasiten-Antikörpern und polyclonalen und/oder monoclonalen mono- oder polygenerischen Anti-Hefen- und/oder Anti-Bakterien- und/oder Anti-Schimmelpilze- und/oder Anti-Parasiten-Antikörpern ausgewählt sind.

6. Verfahren nach Anspruch 5, dadurch **gekennzeichnet**, daß die verwendeten Antikörper durch einen geeigneten Fluorochrom fluoreszierend gemacht werden.
